# EUROPEAN PATENT SPECIFICATION

(11) **EP 4 072 502 B1**
(45) Date of publication and mention of the grant of the patent: **31.01.2024**
(21) Application number: 20820216.8
(22) Date of filing: 10.12.2020
(51) Int. Cl.: A61J 15/00, A61M 25/02

(54) **EXTERNAL RETAINING DEVICE FOR A CATHETER**
EXTERNE HALTEVORRICHTUNG FÜR EINEN KATHETER
DISPOSITIF DE RETENUE EXTERNE POUR UN CATHÉTER

(30) Priority: 11.12.2019 WO PCT/EP2019/084716
(43) Date of publication of application: 19.10.2022
(73) Proprietor: N.V. Nutricia, 2712 HM Zoetermeer (NL); RAUMEDIC AG, 95233 Helmbrechts (DE)
(72) Inventor: VAN KINDEREN, Sencha, 3584 CT Utrecht (NL); WOLKENSTÖRFER, Reinhold, 3584 CT Utrecht (NL); PRESCHER, Jörg, 97729 Ramsthal (DE); MORETH, Daniel, 95028 Hof (DE)
(74) Representative: Nederlandsch Octrooibureau
(86) International application number: PCT/EP2020/085568
(87) International publication number: WO 2021/116302

(56) References cited:
- WO-A1-00/48658
- WO-A1-01/68180
- WO-A2-03/092781
- US-A1- 2015 038 912
- US-A1- 2017 043 131
- US-B1- 6 482 183

## Description

### Field of the invention

The present invention relates to an external retaining device (ERD) for use with a catheter, such as a Percutaneous Endoscopic Gastrostomy (PEG) tube or Gastrostomy tube (G-tube). The invention further relates to a combination of an ERD with a PEG or G-tube and a method of manufacturing an ERD.

### Background art

An External Retaining Device (ERD) is used in the medical field for securing a section of a catheter to remain outside a human body. The invention concerns in particular an ERD for catheters introduced into the stomach for the direct feeding of a patient who cannot be fed by oral intake. The catheter is hence placed through an opening in the abdomen or stoma, into the stomach and the ERD placed around the catheter at the exit from the abdomen. Such catheters used as feeding tubes include Percutaneous Endoscopic Gastrostomy (PEG) tubes mostly produced out of polyurethane and Gastrostomy tubes (G-tubes), which may be made of silicone rubber, being a common polymer inert to body fluids.

The formation of a stoma will take place according to the directions of the medical professional and in light of the recommendations of the manufacturer of the relevant products used. In most cases, PEG tubes are first introduced inside the stomach through the mouth and the oesophagus with the help of a lighted endoscope to ensure their correct positioning in the stomach, wherein a wound, also defined as stoma, is cut at the correct position in the stomach through the abdominal wall. A distal end of the PEG tube is held in place inside the stomach by a disk shaped plate, referred to as a bumper which is larger than the stoma and thus acts as a retaining element for the catheter against the stomach wall. The proximal end of the PEG tube is pulled out of the body through the stoma and retained using the ERD.

Once placed, a PEG tube must be treated with great care, while the stoma heals. The initial period is particularly critical as the wound will be sensitive and subject to considerable exudate. After a period of one or two weeks, the stoma may become stabilised. A PEG tube may remain in use for an extended period of time, subject to the manufacturer's or physician's recommendations and judgement.. If it needs to be replaced, then a G-tube may be inserted through the stoma as this can be achieved without the requirement of an endoscopic procedure.

An ERD is used on the protruding end of the catheter to prevent it from migrating back into the stomach. The bumper prevents it from migrating out of the stomach. Particularly during the first ten days after first placement of the catheter, the stoma must be cleaned and inspected on at least a daily basis. This requires the tube to be released from the ERD and be pushed back slightly to free the bumper so that the tube may be rotated on its axis. In the closed condition of the ERD, the catheter should not move up and down relative to the stoma, as it could create unnecessary and painful friction with the stoma. In this aim it is a requirement for the comfort of the patient to have an ERD that provides good holding forces for retaining the protruding end of the catheter. However, it should also be possible, at any time, to clean the skin around the stoma and the ERD to prevent infection. Hence it is also required to have an ERD that can be easily displaced along the catheter away from the skin, in order to perform proper cleaning. Ideally the placement of the ERD around the catheter and the moving of the ERD along the catheter should be possible without pain for the patient and with ease for the patient and/or the medical staff. It should also be taken into account that each catheter may have a different diameter and/or rigidity. Additionally the ERD should make sure the catheter cannot slide uncontrollably out of the device. It should also be possible to thoroughly clean the ERD itself and inaccessible recesses or cavities should be avoided.

Various ERD designs are known in the prior art, such as shown in prior art document EP0648512B1, however none provides all of the aforementioned advantages. EP0648512B1 describes a bracket for securing a flexible hose protruding from the interior of an organ of the human body, in particular the stomach, through an opening in the abdominal wall to a location outside the body. The bracket comprises a clamping part clampingly receiving the hose, a bend piece connecting through roughly a right angle to the clamping part, and a fixing part intended for placing on the skin, in which said clamping part runs roughly parallel to said fixing part and consists of successive flexible cylindrical sections. A drawback of the known device is that, the flexible hose can easily be incorrectly placed by the user and/or pulled out of the bracket.

Another device is shown US 6 482 183 B1, which has a base plate that can be of a soft elastic material and a pivotally hinged securing element that can be folded and pressed down into a closed position to partially encircle a PEG within a channel. The securing element and its hinge are formed of a relatively hard plastic and the hinge is positioned at a distance from the channel.

A further ERD is shown in WO 00/48658, having pivoting clip elements that can be molded with a body portion. The clip elements can flex into and out of engagement with a tube but do not engage together. Other devices for holding catheters, tubes and connectors on the body are known from WO 0168180, WO 03/092781, US 2017/043131 and US 2015/038912. These devices are not however suitable for retaining an ERD, at least due to the absence of a guiding part for guiding a catheter as it exits a stoma.

It would be desirable to provide a device in which this and other drawbacks are at least partially overcome.

### Summary of the invention

To this end, according to a first aspect, the present invention provides an ERD for securing a catheter exiting a stoma, as further disclosed in claim 1.

The ERD comprises a base plate for engagement with the abdomen of a patient, a guiding part comprising a concave channel open to the top side thereof, for guiding the catheter from the base plate through a first curved section of the concave channel to a substantially parallel position relative to the base plate in a second section of the concave channel and a fixation part. The fixation part has an open state in which a catheter may be introduced into the channel and a closed state for encircling and fixing the catheter within the second section of the guiding part. The fixation part comprises a clip closure comprising two interlocking elements and a hinge therebetween, wherein at least one of the elements comprises rigid polymeric material and at least the base plate and the hinge comprise an elastomeric polymer.

According to the invention, the clip closure can provide a secure lock to block the catheter within the concave channel of the guiding part. Preferably the clip closure should be adequately secure such that it cannot be opened by a patient pulling the catheter in an uncontrolled manner. Hence the catheter can be prevented from being pulled out of the ERD. In particular, since the catheter will be fully encircled by the fixation part, there can be no chance of accidental escape of the catheter due to flexing of the material. In this context, encircle is intended to denote that the fixation part forms a closed loop covering 360 degrees and defining a tubular channel, which can surround and retain the catheter. It does not imply any particular circular shape to the channel. Additionally the curved guiding part of the ERD smoothly guides the catheter through a 90 degree angle, avoiding the catheter to become kinked, thus blocking the inner lumen and facilitating discreet wearing beneath clothing without requiring additional fastening. Locking by means of a clip in this manner may be relatively easy and patient-friendly. Furthermore the use of an elastomeric polymer provides a soft base plate for contact with the skin near the stoma and an elastomeric hinge that is robust and can be opened and closed an almost infinite number of times. In this sense, the elastomeric hinge is similar to a living hinge although the term living hinge is usually used to refer to a plastic material that has been rendered flexible at a hinge location in a moulding process, The present elastomeric hinge is inherently both flexible and elastic in that it can also stretch. The elastomeric hinge is part of the fixation part and may allow pivoting about an axis lying parallel to the second section of the concave channel.

In certain embodiments, the clip closure may comprise just one rigid polymeric element, which engages with another flexible closure part. This may be in the form of a strap for engaging with a rigid pin or barb. In a preferred embodiment the clip closure may comprise two interlocking rigid polymer elements. The clicking engagement of two rigid elements may provide a relatively strong lock to securely retain the catheter within the concave channel. It can also provide certainty for the user that locking has occurred due to an audible clicking sound. The engaging surfaces of the rigid polymer elements can be easily adapted according to the level of engagement required.

In an embodiment, at least one of the two interlocking rigid polymer elements may be rotatable through an angle of at least 60° by rotation of the hinge from the closed state to the open state. A large opening angle between the two interlocking rigid polymer elements in the open state of the clip closure can provide sufficient width to freely lay the catheter down into the concave channel, removing any obstacle that would demand to exert a downward force onto the base plate, and hence onto the painful stoma of the patient.

In an embodiment, the two interlocking rigid polymer elements only partially encircle the catheter in the closed state. The extent of their circumferential coverage will depend on the amount of the circumference of the catheter that they need to enclose in order to perform their function. On the one hand, this may depend on the nature of the clip closure and the way in which the elements engage together. On the other hand, it will also depend on the engagement between the rigid polymeric material and the elastomeric polymer and the extent to which they must be engaged e.g. by overmoulding as further discussed below. The amount of rigid material compared to the amount and nature of the elastomeric material will also determine the overall resilience of the engagement with the catheter. In an embodiment, the two interlocking rigid polymer elements may cover from 5 to 360 degrees of the circumference, preferably between 90 and 355 degrees and most preferably, between 220 and 330 degrees.

The skilled person will recognise that many different locking arrangements can be contemplated for engaging the clip closure. Most preferably, engagement will be relatively easy e.g. with a single hand, while disengagement may be more difficult, e.g. requiring two hands. In this manner, a patient may be prevented from accidentally releasing the clip closure. Furthermore, closure should preferably be achieved with minimum force exerted on the body of the patient, which could cause pain or irritate a stoma that is in the process of healing. The ERD may be provided with suitable external ergonomic form and indicators to encourage correct placement of the fingers in order to accomplish correct manipulation. In one embodiment the two interlocking rigid polymer elements may comprise a slot and a barbed finger that engage together to lock the fixation part in the closed state. The snapclosure of the two engaging elements can provide a strong lock that can nevertheless be relatively easily opened.

According to the invention, the body of the ERD comprising the base plate, guiding part and resilient portions of the fixation part are all integrally formed as a single device from the elastomeric material. The remaining rigid, interlocking parts are integrated therewith in the moulding or manufacturing process to achieve a one-component ERD. The absence of loose parts in the ERD reduces clean room operations for assembly, limits the amount of stock required and avoids loss of individual small pieces by the medical staff or the patient and any associated risks of swallowing or choking. It also may make the operation of the device easier and more self-evident and ensures that the device is easy to keep clean, since there are no joints or crevices between different components.

The rigid polymeric material is at least in part overmoulded by the elastomeric polymer. The overmoulding by the elastomeric polymer minimizes the exposure of rigid polymer to the hand and skin of the patient, providing a soft layer for comfort and grip, while ensuring larger friction and holding forces for the catheter inside the channel. Overmoulding is also a preferred way of engaging the materials together as it may ensure a greater contact surface over which the rigid material and the elastomeric materials may bond together. In one embodiment, the overmoulding ensures that the catheter is fully encircled by the elastomeric polymer. In the case that the elastomeric polymer is in contact with the catheter around or almost around its full circumference, the frictional retention will be maximised.

The rigid polymeric material may be any suitable polymer that can provide the mechanical and material properties required of the device, in particular those related to the overmoulding process. The interlocking elements may also be formed of a mixture of such materials, e.g. a layered product with a core of a first material providing mechanical properties and an exterior of another material comprising surface or compatibility properties. If two or more interlocking elements of rigid polymeric material are provided, they may be of the same or different materials. In an embodiment the rigid polymeric material comprises a polyester, preferably polybutylterephthalate (PBT), including its equivalents. Polyester material, in particular PBT is known for providing good cross-linking with silicone during injection overmoulding and therefore a strong chemical fixation.

In the present context, rigid is intended to denote that the material is relatively more rigid than the elastomeric material. In particular, the material should be sufficiently rigid to form a clip type connection that will be able to withstand the forces imposed on the contact areas available for clipping. Examples of rigid polymeric materials may include not only the PBT mentioned above but also polamides. Polyolefins and other polymeric materials having a flexural modulus of greater than 500 MPa.

In an embodiment, the elastomeric polymer comprises silicone rubber. Other thermoplastic elastomers (TPE) and thermoplastic polyurethanes (TPU) may also be used including copolymers thereof. The elastomeric polymeric material is chosen to provide flexibility and softness to the device and a strongly fixed overmoulded layer. Silicone rubber, otherwise referred to as silicone is a most preferred material. It is a thermosetting elastomer that can be moulded from liquid silicone rubber and cured and which has showed itself to be ideal for use in medical applications. The flexibility of the elastomeric polymer may be a number of orders of magnitude lower than that of the rigid polymeric material. In an example, the elastomeric material may have a flexural modulus that is less than 10% of that of the rigid polymeric material.

In an embodiment, a closing part closes the concave channel at an upper side, distant from the base plate. The closing part may also be manufactured of the same elastomeric polymer as the hinge. In the closed state of the device the catheter may be in contact with the soft elastic closing part thus providing better holding forces than rigid polymeric materials while allowing the channel to accommodate tubes of various diameters due to the elasticity of the closing part. The hinge allows pivoting of the closing part with respect to the base plate about an axis that is generally parallel to the second section of the concave channel.

In an embodiment, the closing part is flexible or resilient, such that catheters of a radius up to 30% larger than a radius of the concave channel can be retained in the fixation part. The flexibility of the closing part allows one single ERD to accommodate and lock catheters of different diameters. For PEG and G-tubes, diameters are usually given in French or Charriere values, being 3 times the diameter of the tube in mm. For the ERD as presently disclosed, tubes for instance of CH14 to CH16, may be received in a channel of diameter 5 mm. This may give greater flexibility in terms of stock management and assembly operations. In general, each ERD may be suitable for reception of 3 different CH sizes. The flexibility of the closing part also ensures that other elements may be captured or retained within the fixation part. In certain embodiments, it may be desirable to use the ERD to retain the proximal end of the catheter in place e.g. by capturing a loop of a connector within the fixation part.

In an embodiment, the base plate comprises a passage for friction-free engagement with the catheter. The friction-free engagement of the catheter with a passage that is at least equal to the outer diameter of the catheter, allows the user to displace the ERD with ease along the catheter to clean the stoma. Preferably, the passage is sufficiently larger than the intended catheter that no interference is experienced during movement. In general, it will be understood that the passage will be an enclosed passage i.e. an opening through the base plate. In other embodiments, it is however not excluded that the base plate comprises a passage that is open to an end or side of the base plate, such that the base plate may be engaged laterally without requiring it to be threaded over the end of the catheter.

In another embodiment, a plane of opening of the fixation part is at an angle to the plane of the base plate, preferably 20 degrees to 90 degrees. This tilted plane allows a user to open and close the fixation part with solely two fingers and with enough room to place one finger between the base plate and the closing element. It also ensures that forces exerted during opening and closing are directed at least partially laterally in a direction parallel to the base plate, rather than in a direction downwards onto the stoma.

According to a second aspect, the present invention also discloses a method of manufacturing an external retention device for securing a catheter exiting a stoma, as further disclosed in claim 12, comprising providing first and second interlocking rigid polymer elements; arranging said elements in a mould; overmoulding an elastomeric polymer over the first and second interlocking rigid polymer elements to form a concave channel having a curved first section, a straight second section, a hinge and a base plate.

In an embodiment, the overmoulding takes place with the first and second interlocking rigid polymer elements in an open state, wherein the closing part defines an angle of 60 degrees to 180 degrees between the interlocking rigid polymer elements during overmoulding. The moulding of the ERD with the hinge defining an angle of at least 60 degrees between the interlocking elements, preferably in an open state with an angle of 180 degrees, allows an easier and cost-effective manufacturing process.

In an embodiment, the method of manufacturing further comprises post-curing the external retention device in a closed state, wherein the clip closure is closed. By post-curing the external retaining device in a closed state, the fixing element is naturally biased to the closing position, making sure that the clip can be closed with less effort and reduced rotation of the closing part. The manufacture also supports the closing of the clip closure with just one hand. Alternatively, it may be desirable to post-cure the clip closure in the open position, so that it is readily visible if the clip has accidentally opened. Still further, a semi-open position may be preferred so that insertion of the tube is easily achieved while movement from the post-curing position to the closed condition can still be accomplished with one hand.

Depending on the intended method of use, the ERD may be provided with suitable external ergonomic features and indicators to encourage correct manipulation. These may include surface texture or relief on either the elastomeric polymer e.g. of the fixation portion or the rigid polymer elements.

### Short description of drawings

The present invention will be discussed in more detail below, with reference to the attached drawings, in which:
Figs. 1A and 1B show a schematic view of an external retaining device in use for feeding a patient in accordance with the present invention;
Figs. 2A and 2B show perspective views of the external retaining device in accordance with the present invention with the device in open (a) and closed (b) states;
Fig. 3 shows an exploded view of the external retaining device in accordance with the present invention with the device in open state
Fig. 4 shows a top view of the external retaining device in accordance with the present invention in closed state
Fig. 5 shows a front view of the external retaining device in accordance with the present invention in closed state

### Description of drawings

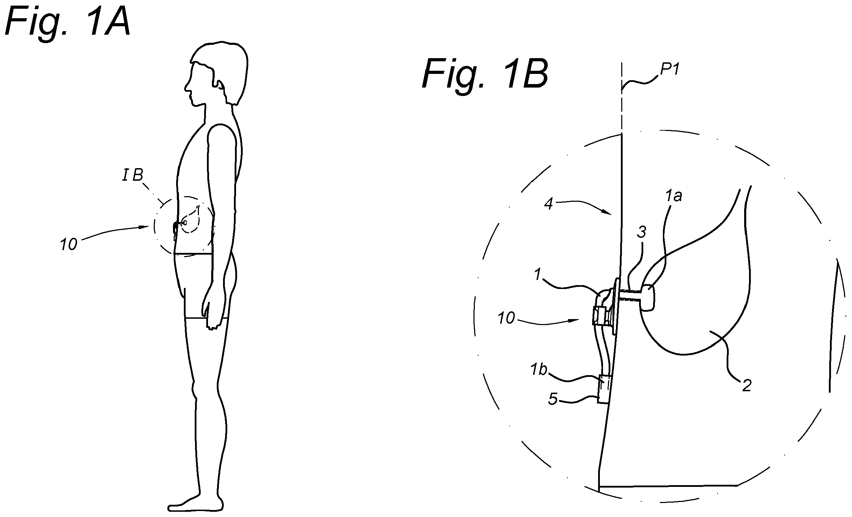

Fig. 1A shows a schematic view of an exemplary External Retaining Device (ERD) 10 in use for feeding a patient. Further detail is shown in Fig. 1B. An exemplary catheter 1 is introduced into the stomach 2 of a patient to be fed and protrudes outside of the patient's body, through a stoma 3 in the abdomen 4. The exemplary catheter 1 is a polyurethane PEG with a diameter of CH14 although it will be understood that the following description applies equally to G-tubes and PEG tubes of all relevant materials and sizes. Such feeding tubes can present various diameters such as from CH4 to CH20 to match the stoma size and the required feeding. The catheter 1 comprises a distal end 1a with a bumper to maintain the distal end 1a inside the stomach while the proximal end 1b of the catheter 1 protrudes through the stoma 3. Although reference is given to a stoma and the abdomen, this is not intended to be in any way limiting on the device as claimed and alternative placements may be contemplated.

The ERD 10 is placed along plane P1 on the skin of the abdomen 4 over the stoma 3 and receives portion of the catheter 1 that protrudes through the stoma 3 , to prevent it from sliding back into the stomach 2. Devices are produced in other sizes to receive tubes of other diameters. The proximal end 1b of the catheter 1 that extends beyond the ERD 10 is provided with a connector (not shown) that will connect to syringes and feeding sets. This is taped back onto the abdomen to keep it from protruding underneath the user's clothes. The proximal end 1b may also be engaged with the ERD as will be further discussed below.

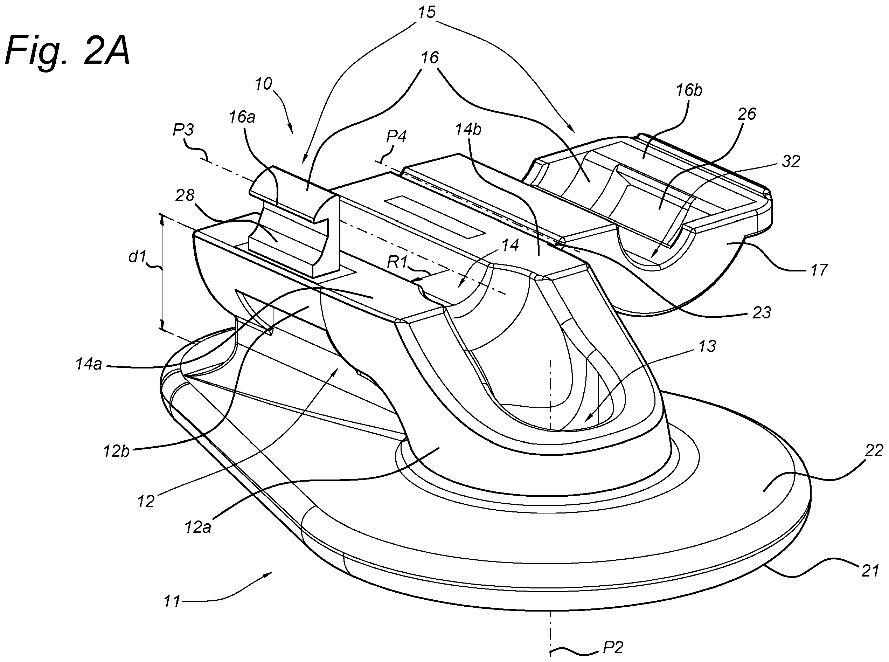

Fig. 2A shows a perspective view of the ERD 10 according to the present invention with the device in open state.

As can be seen, the depicted ERD 10 is a one-component device comprising integrally formed elements, obtained by a moulding process including a base plate 11, a guiding part 12 and a fixation part 15. The base plate 11 has a lower surface 21 and an upper surface 22 defining a thickness of the base plate 11 with the lower and upper surfaces 21, 22 connected by a passage 13 having a central axis P2. The base plate 11 is made of soft and elastic silicone rubber. The guiding part 12 comprises a first section 12a with a semi-circular concave channel 14 open to the top side thereof, extending upwards from the passage 13 in a curve relative to the base plate 11. The guiding part 12 further comprises a second section 12b, contiguous to the first section 12a, where the same concave channel 14 extends parallel to the base plate 11, at a vertical distance d₁ offset from the base plate 11. The concave channel 14 extends from the first section 12a to the second section 12b, along a longitudinal axis P3, between two planar surfaces, namely a clip side surface 14a and a hinge side surface 14b. A radius of the channel 14 in the first section 12a varies from the passage 13 to the second section 12b, where the radius is fixed to a value R1. The angle between P2 and P3 at the second section is 90 degrees.

The fixation part 15 is located in the second section 12b of the guiding part 12 and surrounds or encircles the concave channel 14. It includes a clip closure 16 a closing part 17 and a hinge 23. The closing part 17 comprises silicon rubber and is connected via the hinge 23 at the hinge side surface 14b of the concave channel 14 so as to be selectively rotatable by 180° around an axis P4. In this open state of the ERD 10, the closing part 17 is defined to be positioned at angle 0. The closing part 17 includes a second channel 32 of similar size to the semi-circular concave channel 14 in the second section 12b. The clip closure 16 consists of first and second interlocking elements 16a, 16b, the first element 16a protrudes from the clip side surface 14a of the concave channel 14 and comprises a barbed finger 28. The second element 16b is provided on the closing part 17 and comprises a slot 26. The barbed finger 28 is receivable in the slot 26 when the fixation part 15 is in a closed state. The interlocking elements 16a, 16b of the clip closure are made of polybutylene terephthalate (PBT) and are embedded in silicon rubber which forms the remainder of the fixation part 15. PBT is known for its good cross-linking with silicone during injection moulding, resulting in an improved adhesion of the overmoulded silicone, as disclosed in WO2017216068A1. The silicone rubber also provides a soft cover to the rigid PBT.

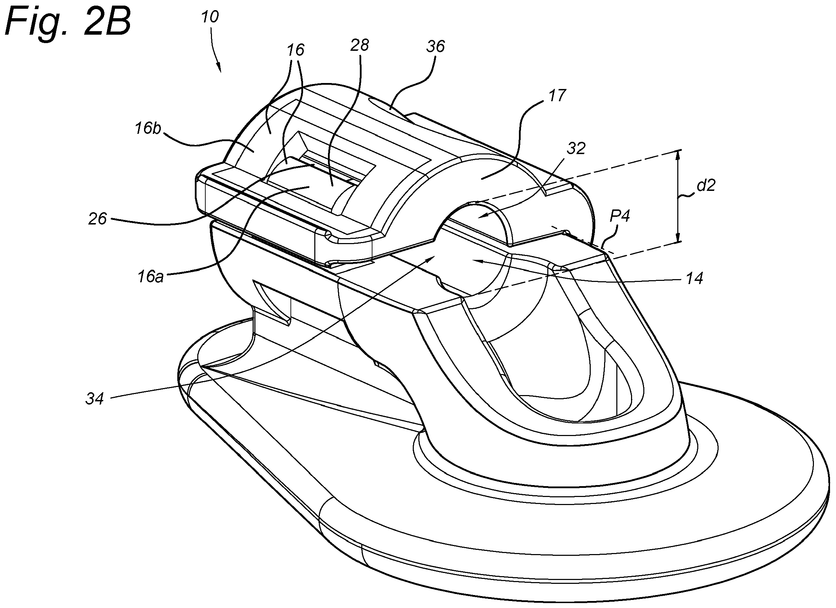

Fig. 2B shows the same view of the ERD 10 with the device in a closed state. In the closed state the closing part 17 is positioned at an angle of 180° relative to the open state, after rotation of the closing part 17 comprising the first interlocking element 16a to engage with the second interlocking element 16b. In this position the barbed finger 28 is thus engaged in the slot 26. The closure of the clip closure 16 positions the second channel 32 of the closing part 17 above the semi-circular concave channel 14, to form a tubular channel 34 of diameter d₂.

The fixation part 15 thus comprises rigid plastic overmoulded with silicone rubber to form the tubular channel 34. Fig. 2B also shows a depression 36 on an upper side of the closing part 17, which indicates a preferred location for a user's finger.

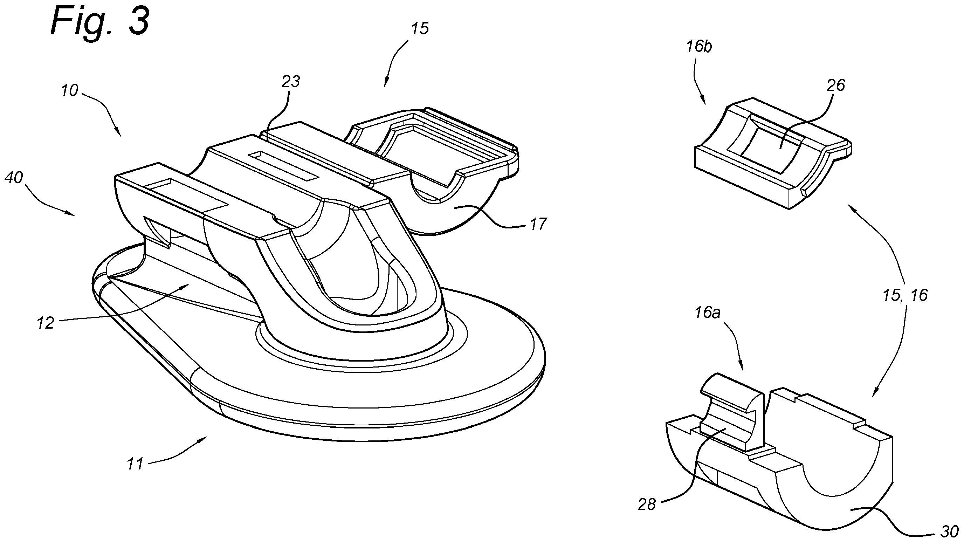

Fig. 3 shows an exploded view of the ERD 10 according to the present invention with the device in open state. The main body 40 of the ERD 10 comprising the base plate 11, the guiding part 12 and the fixation part 15, including the closing part 17 and the hinge 23 are all made in one piece of soft and elastic silicone rubber. They are shown separately from the interlocking elements 16a, 16b of the clip closure 16, which are made of PBT and comprise the barbed finger 28 and the slot 26. As can better be seen in this view, the first interlocking element 16a comprises a curved, channel section 30 from which the barbed finger 28 extends. The second interlocking element 16b is also generally curved with a similar curvature to the channel section 30 and the concave channel 14.

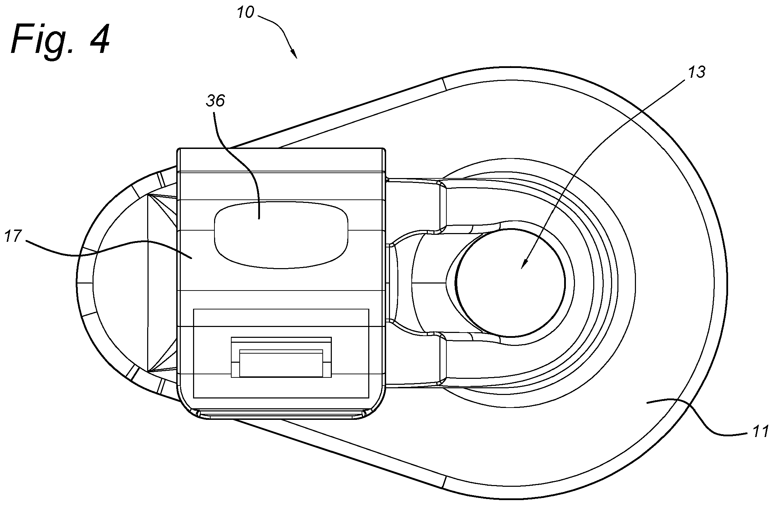

Fig. 4 shows a top view of the ERD 10 according to the invention with the device in closed state. The passage 13 can be seen to have a substantially circular shape. Also visible in this view is the depression 36 on the upper side of the closing part 17.

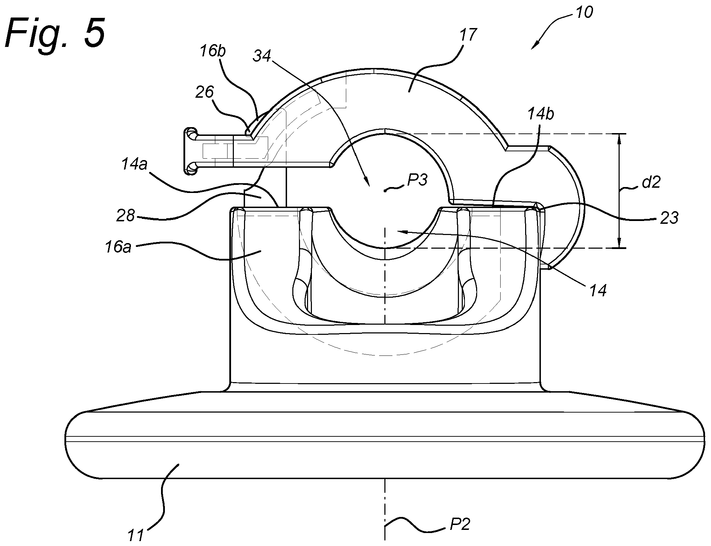

Fig. 5 shows a front view of the ERD 10 according to the present invention with the device in closed state. The closing element 17 closes the top side of the concave channel 14 by fitting of the slot 26 over the barbed finger 28, whereby a catheter (not shown) can be locked within the tubular channel 34. This can be achieved by applying a squeezing pressure laterally to the ERD 10, whereby a user's finger engages the depression 36. In Fig. 5, it can also be appreciated that the clip side surface 14a and hinge side surface 14b lie in the same plane which is referred to as the plane of opening of the fixation part 15. In this embodiment, the plane of opening is parallel to the base plate 11. It will be understood that in alternative embodiments, the plane of opening may be angled with respect to the base, such that the hinge 23 is lowered. This may ensure that a force required to close the fixation part is directed sideways rather than downwards.

Inadvertent opening of the ERD 10 is also avoided by the firm engagement between the barbed finger 28 and the slot 26. To release this engagement, a user must squeeze the ERD 10 laterally, whereby the closing element 17 is pushed to the left in Figure 5, with respect to the lower portion of the fixation portion 15. This causes the second interlocking element 16b to also move to the left with respect to the first interlocking elements 16a. This movement is at least partially facilitated by the elastomeric material of the closing element 17 and the hinge 23 and causes relese of the barbed finger 28 from the slot 26. Again, this action takes place without downwards pressure on the ERD 10, which could cause pain or discomfort to the patient. Due to the memory effect of post curing the ERD 10 in an open position, the closing element 17 will automatically pop up to the fully open position.

Also visible in shadow in Fig. 5 is the extent by which the interlocking elements 16a, 16b encircle the tubular channel 34. In the illustrated embodiment, the first interlocking element 16a subtends an angle of around 180 degrees, extending over the full extent of the concave channel 14. The second interlocking element subtends an angle of around 90 degrees or around half of the closing element 17. In total, around 270 degrees of the circumference of the tubular channel is enclosed by the rigid polymeric material of the interlocking elements 16a, 16b, while the remaining 90 degrees, including the hinge 23 comprises only elastomeric polymer material. This balance between rigid and elastomeric materials and the resilience of the elastomeric material determines the amount of flexibility or resilience in the fixation part 15.

## Claims

1. An external retention device (10) for securing a catheter (1) exiting a stoma (3), comprising:
a base plate (11) for engagement with the abdomen (4) of a patient;
a guiding part (12) comprising a concave channel (14) open to the top side thereof, for guiding the catheter (1) from the base plate (11) through a first curved section (12a) of the concave channel (14) to a substantially parallel position relative to the base plate (11) in a second section (12b) of the concave channel (14);
a fixation part (15) having an open state and a closed state for encircling and fixing the catheter (1) within the second section (12b) of the guiding part (12), the fixation part (15) comprising a clip closure (16) comprising two interlocking elements (16a, 16b) and a hinge (23) therebetween, wherein at least one of the interlocking elements (16a, 16b) comprises rigid polymeric material and at least the base plate (11) comprise an elastomeric polymer, **characterized in that** the hinge (23) also comprises an elastomeric polymer, the base plate (11), guiding part (12) and fixation part (15) are integrally formed as a single device, and the rigid polymeric material is at least in part overmolded by the elastomeric polymer.

2. The external retention device according to claim 1, wherein both of the interlocking elements (16a, 16b) of the clip closure (16) comprise rigid polymeric material.

3. The external retention device according to claim 1 or claim 2, wherein at least one of the two interlocking elements (16a, 16b) is rotatable through an angle of at least 60° by rotation of the hinge (23) to the open state.

4. The external retention device according to claim 2 or claim 3, wherein the rigid polymer material only partially encircles the catheter (1) in the closed state, preferably covering between 90 and 330 degrees.

5. The external retention device according to any one of claims 2-4, wherein the two interlocking elements (16a, 16b) comprise a slot (26) and a barbed finger (26) that interlock together.

6. The external retention device according to any one of the preceding claims, wherein the rigid polymeric material is a polyester, preferably polybutylterephthalate (PBT).

7. The external retention device according to any one of the preceding claims, wherein the elastomeric polymer comprises silicone, polyurethane or copolymers thereof.

8. The external retention device according to any one of the preceding claims, wherein a closing part (17) closes the concave channel (14) at a top side thereof, distant from the base plate (11) and a first (16a) of the two interlocking elements is provided on the closing part (17).

9. The external retention device according to claim 8, wherein the closing part (17) also comprises the elastomeric polymer and is sufficiently resilient such that catheters having a radius, preferably up to 30%, larger than a radius of the concave channel (14) can be retained in the fixation part (15).

10. The external retention device according to any one of the preceding claims, wherein the base plate (11) comprises a passage (13) for friction free engagement with the catheter (1).

11. The external retention device according to any one of the preceding claims, wherein a plane of opening of the fixation part (15) is at an angle to the plane of the base plate (11), preferably at an angle of from 20 degrees to 90 degrees.

12. A method of manufacturing the external retention device (10) of claim 1 for securing a catheter (1) exiting a stoma (3), comprising:
providing a clip closure (16) comprising first (16a) and second (16b) interlocking rigid polymer elements;
arranging said elements (16a, 16b) in a mould;
overmoulding an elastomeric polymer over the first (16a) and second (16b) interlocking rigid polymer elements to form a concave channel (14) having a curved first section (12a), a straight second section (12b), a hinge (23) and a base plate (11), wherein the first (16a) and second (16b) interlocking rigid polymer elements can interlock together to encircle and fix the catheter (1) within the second section (12b).

13. The method according to claim 12 wherein moulding takes place with the first (16a) and second (16b) interlocking rigid polymer elements in an open state, at an angle of 160 degrees to 180 degrees relative to their closed state.

14. The method according to claim 12 or claim 13 further comprising post-curing the external retention device (10) in a closed state in which the clip closure (16) is closed or in a half open state.

15. The method according to claim 12 or claim 13 further comprising post-curing the external retention device (10) in an open position, such that it is visible if the clip closure (16) has opened.

## Patentansprüche

1. Externe Haltevorrichtung (10) zum Befestigen eines Katheters (1), der aus einem Stoma (3) austritt, die Folgendes umfasst:
eine Basisplatte (11) zum Eingriff mit dem Unterleib (4) eines Patienten;
einen Führungsteil (12), der einen konkaven Kanal (14) umfasst, der zu seiner Oberseite offen ist, um den Katheter (1) von der Basisplatte (11) durch einen ersten gekrümmten Abschnitt (12a) des konkaven Kanals (14) zu einer im Wesentlichen parallelen Position relativ zu der Basisplatte (11) in einem zweiten Abschnitt (12b) des konkaven Kanals (14) zu führen;
einen Befestigungsteil (15) mit einem offenen Zustand und einem geschlossenen Zustand zum Umschließen und Befestigen des Katheters (1) innerhalb des zweiten Abschnitts (12b) des Führungsteils (12), wobei das Befestigungsteil (15) einen Clipverschluss (16) umfasst, der zwei Verriegelungselemente (16a, 16b) und ein Scharnier (23) dazwischen umfasst, wobei mindestens eines der Verriegelungselemente (16a, 16b) ein starres Polymermaterial umfasst und zumindest die Basisplatte (11) ein Elastomerpolymer umfasst, **gekennzeichnet dadurch, dass** das Scharnier (23) auch ein Elastomerpolymer umfasst, die Basisplatte (11), der Führungsteil (12) und der Befestigungsteil (15) einstückig als eine einzige Vorrichtung gebildet sind und das starre Polymermaterial zumindest teilweise durch das Elastomerpolymer überformt ist.

2. Externe Haltevorrichtung nach Anspruch 1, wobei beide Verriegelungselemente (16a, 16b) des Clipverschlusses (16) ein starres Polymermaterial umfassen.

3. Externe Haltevorrichtung nach Anspruch 1 oder Anspruch 2, wobei mindestens eines der beiden Verriegelungselemente (16a, 16b) durch Drehung des Scharniers (23) in den offenen Zustand um einen Winkel von mindestens 60° gedreht werden kann.

4. Externe Haltevorrichtung nach Anspruch 2 oder Anspruch 3, wobei das starre Polymermaterial nur teilweise den Katheter (1) in dem geschlossenen Zustand umschließt, wobei es vorzugsweise zwischen 90 und 330 Grad abdeckt.

5. Externe Haltevorrichtung nach einem der Ansprüche 2-4, wobei die beiden Verriegelungselemente (16a, 16b) einen Schlitz (26) und einen mit Widerhaken versehenen Finger (26) umfassen, die ineinandergreifen.

6. Externe Haltevorrichtung nach einem der vorhergehenden Ansprüche, wobei das starre Polymermaterial ein Polyester ist, vorzugsweise Polybutylterephthalat (PBT).

7. Externe Haltevorrichtung nach einem der vorhergehenden Ansprüche, wobei das Elastomerpolymer Silikon, Polyurethan oder Copolymere davon umfasst.

8. Externe Haltevorrichtung nach einem der vorhergehenden Ansprüche, wobei ein Verschlussteil (17) den konkaven Kanal (14) an seiner von der Basisplatte (11) entfernten Oberseite verschließt und ein erstes (16a) der beiden Verriegelungselemente an dem Verschlussteil (17) vorgesehen ist.

9. Externe Haltevorrichtung nach Anspruch 8, wobei der Verschlussteil (17) auch das Elastomerpolymer umfasst und ausreichend elastisch ist, so dass Katheter mit einem Radius, der vorzugsweise bis zu 30 %, größer als ein Radius des konkaven Kanals (14) ist, in dem Befestigungsteil (15) gehalten werden können.

10. Externe Haltevorrichtung nach einem der vorhergehenden Ansprüche, wobei die Basisplatte (11) einen Durchgang (13) zum reibungsfreien Eingriff mit dem Katheter (1) umfasst.

11. Externe Haltevorrichtung nach einem der vorhergehenden Ansprüche, wobei eine Öffnungsebene des Befestigungsteils (15) in einem Winkel zu der Ebene der Basisplatte (11) liegt, vorzugsweise in einem Winkel von 20 Grad bis 90 Grad.

12. Verfahren zum Herstellen der externen Haltevorrichtung (10) nach Anspruch 1 zum Befestigen eines Katheters (1), der aus einem Stoma (3) austritt, das Folgendes umfasst:
Bereitstellen eines Clipverschlusses (16), der ein erstes (16a) und zweites (16b) ineinandergreifendes starres Polymerelement umfasst;
Anordnen der besagten Elemente (16a, 16b) in einer Form;
Überformen eines Elastomerpolymers über das erste (16a) und zweite (16b) ineinandergreifende starre Polymerelement, um einen konkaven Kanal (14) mit einem gekrümmten ersten Abschnitt (12a), einem geraden zweiten Abschnitt (12b), einem Scharnier (23) und einer Basisplatte (11) zu bilden, wobei das erste (16a) und zweite (16b) ineinandergreifende starre Polymerelement ineinandergreifen können, um den Katheter (1) innerhalb des zweiten Abschnitts (12b) zu umschließen und zu befestigen.

13. Verfahren nach Anspruch 12, wobei das Formen mit dem ersten (16a) und zweiten (16b) ineinandergreifenden starren Polymerelement in einem offenen Zustand in einem Winkel von 160 Grad bis 180 Grad relativ zu ihrem geschlossenen Zustand erfolgt.

14. Verfahren nach Anspruch 12 oder Anspruch 13, das ferner das Nachhärten der externen Haltevorrichtung (10) in einem geschlossenen Zustand, in dem der Clipverschluss (16) geschlossen ist, oder in einem halboffenen Zustand umfasst.

15. Verfahren nach Anspruch 12 oder Anspruch 13, das ferner das Nachhärten der externen Haltevorrichtung (10) in einer offenen Position umfasst, so dass es sichtbar ist, wenn sich der Clipverschluss (16) geöffnet hat.

## Revendications

1. Dispositif de rétention externe (10) pour sécuriser un cathéter (1) sortant d'une stomie (3), comprenant :
une plaque de base (11) pour l'engagement avec l'abdomen (4) d'un patient ;
une partie de guidage (12) comprenant un canal concave (14) ouvert sur le côté supérieur de cela, pour guider le cathéter (1) depuis la plaque de base (11) à travers une première section incurvée (12a) du canal concave (14) jusqu'à une position sensiblement parallèle par rapport à la plaque de base (11) dans une deuxième section (12b) du canal concave (14) ;
une partie de fixation (15) ayant un état ouvert et un état fermé pour encercler et fixer le cathéter (1) dans la deuxième section (12b) de la partie de guidage (12), la partie de fixation (15) comprenant une fermeture à clip (16) comprenant deux éléments de verrouillage (16a, 16b) et une charnière (23) entre eux, où au moins l'un des éléments de verrouillage (16a, 16b) comprend un matériau polymère rigide et au moins la plaque de base (11) comprend un polymère élastomère, **caractérisé en ce que** la charnière (23) comprend également un polymère élastomère, la plaque de base (11), la partie de guidage (12) et la partie de fixation (15) sont formées intégralement comme un dispositif unique, et le matériau polymère rigide est au moins en partie surmoulé par le polymère élastomère.

2. Dispositif de rétention externe selon la revendication 1, où les deux des éléments de verrouillage (16a, 16b) de la fermeture à clip (16) comprennent un matériau polymère rigide.

3. Dispositif de rétention externe selon la revendication 1 ou la revendication 2, où au moins l'un des deux éléments de verrouillage (16a, 16b) peut pivoter d'un angle d'au moins 60° par rotation de la charnière (23) à l'état ouvert.

4. Dispositif de rétention externe selon la revendication 2 ou la revendication 3, où le matériau polymère rigide n'encercle que partiellement le cathéter (1) à l'état fermé, couvrant de préférence entre 90 et 330 degrés.

5. Dispositif de rétention externe selon l'une quelconque des revendications 2 à 4, où les deux éléments de verrouillage (16a, 16b) comprennent une fente (26) et un doigt barbelé (26) qui se verrouillent ensemble.

6. Dispositif de rétention externe selon l'une quelconque des revendications précédentes, où le matériau polymère rigide est un polyester, de préférence du polybutyltéréphtalate (PBT).

7. Dispositif de rétention externe selon l'une quelconque des revendications précédentes, où le polymère élastomère comprend de la silicone, du polyuréthane ou des copolymères de cela.

8. Dispositif de rétention externe selon l'une quelconque des revendications précédentes, où une partie de fermeture (17) ferme le canal concave (14) sur un côté supérieur de cela, éloigné de la plaque de base (11) et un premier (16a) des deux éléments de verrouillage est pourvu sur la partie de fermeture (17).

9. Dispositif de rétention externe selon la revendication 8, où la partie de fermeture (17) comprend également le polymère élastomère et est suffisamment élastique de sorte que les cathéters ayant un rayon, de préférence jusqu'à 30%, plus grand qu'un rayon du canal concave (14) peuvent être retenus dans la partie de fixation (15).

10. Dispositif de rétention externe selon l'une quelconque des revendications précédentes, où la plaque de base (11) comprend un passage (13) pour un engagement sans friction avec le cathéter (1).

11. Dispositif de rétention externe selon l'une quelconque des revendications précédentes, où un plan d'ouverture de la partie de fixation (15) est à un angle par rapport au plan de la plaque de base (11), de préférence à un angle de 20 degrés à 90 degrés.

12. Procédé de fabrication du dispositif de rétention externe (10) selon la revendication 1 pour sécuriser un cathéter (1) sortant d'une stomie (3), comprenant :
fournir une fermeture à clip (16) comprenant un premier (16a) et un deuxième (16b) élément de polymère rigide de verrouillage ;
disposer lesdits éléments (16a, 16b) dans un moule ;
surmouler un polymère élastomère sur le premier (16a) et deuxième (16b) éléments de polymère rigide de verrouillage pour former un canal concave (14) ayant une première section incurvée (12a), une deuxième section droite (12b), une charnière (23) et une plaque de base (11), où le premier (16a) et deuxième (16b) éléments de polymère rigide de verrouillage peuvent se verrouiller ensemble pour encercler et fixer le cathéter (1) dans la deuxième section (12b).

13. Procédé selon la revendication 12, où le moulage a lieu avec le premier (16a) et deuxième (16b) éléments de polymère rigide de verrouillage dans un état ouvert, à un angle de 160 degrés à 180 degrés par rapport à leur état fermé.

14. Procédé selon la revendication 12 ou la revendication 13 comprenant en outre le post-durcissement du dispositif de rétention externe (10) dans un état fermé dans lequel la fermeture à clip (16) est fermée ou dans un état semi-ouvert.

15. Procédé selon la revendication 12 ou la revendication 13 comprenant en outre le post-durcissement du dispositif de rétention externe (10) dans une position ouverte, de sorte qu'il soit visible si la fermeture à clip (16) s'est ouverte.
